(19) **Europäisches Patentamt**
European Patent Office
Office européen des brevets

(11) **EP 1 628 763 B1**

(12) **EUROPÄISCHE PATENTSCHRIFT**

(45) Veröffentlichungstag und Bekanntmachung des Hinweises auf die Patenterteilung:
**12.09.2007 Patentblatt 2007/37**

(21) Anmeldenummer: **04733250.7**

(22) Anmeldetag: **15.05.2004**

(51) Int Cl.:
**B01J 35/02** (2006.01)   **C07C 51/265** (2006.01)

(86) Internationale Anmeldenummer:
**PCT/EP2004/005247**

(87) Internationale Veröffentlichungsnummer:
**WO 2004/103561 (02.12.2004 Gazette 2004/49)**

(54) **DREI- BZW. VIERSCHICHTIGE KATALYSATORSYSTEME ZUR HERSTELLUNG VON PHTHALSÄUREANHYDRID**

THREE-LAYERED OR FOUR-LAYERED CATALYST SYSTEMS FOR PRODUCING PHTHALIC ANHYDRIDE

SYSTEMES CATALYTIQUES A TROIS OU A QUATRE COUCHES, UTILISES POUR PRODUIRE DE L'ANHYDRIDE D'ACIDE PHTALIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.05.2003 DE 10323818**

(43) Veröffentlichungstag der Anmeldung:
**01.03.2006 Patentblatt 2006/09**

(73) Patentinhaber: **BASF Aktiengesellschaft 67056 Ludwigshafen (DE)**

(72) Erfinder:
• **STORCK, Sebastian 68167 Mannheim (DE)**
• **ZÜHLKE, Jürgen 67346 Speyer (DE)**
• **NETO, Samuel 68161 Mannheim (DE)**
• **ROSOWSKI, Frank 68165 Mannheim (DE)**

(56) Entgegenhaltungen:
**EP-A- 1 063 222          WO-A-02/16299**
**DE-A- 19 633 757          DE-A- 19 823 262**
**US-B1- 6 369 240**

**Beschreibung**

Die Erfindung betrifft Katalysatorsysteme zur Herstellung von Phthalsäureanhydrid mittels Gasphasenoxidation von o-Xylol und/oder Naphthalin, sowie ein Verfahren zur Herstellung von Phthalsäureanhydrid unter Verwendung der Katalysatorsysteme.

Phthalsäureanhydrid wird technisch durch katalytische Gasphasenoxidation von o-Xylol oder Naphthalin in Rohrbündelreaktoren hergestellt. Ausgangsmaterial ist ein Gemisch aus einem molekularen Sauerstoff enthaltenden Gas, beispielsweise Luft, und dem zu oxidierenden o-Xylol und/oder Naphthalin. Das Gemisch wird durch eine Vielzahl in einem Reaktor angeordneter Rohre (Rohrbündelreaktor) geleitet, in denen sich eine Schüttung mindestens eines Katalysators befindet. In den letzten Jahren ist man dazu übergegangen, unterschiedlich aktive Katalysatoren schichtweise in der Katalysatorschüttung anzuordnen, wobei sich in der Regel der weniger aktive Katalysator zum Gaseintritt hin in der ersten, obersten Katalysatorschicht und der aktivere Katalysator zum Gasaustritt hin in der letzten, untersten Katalysatorschicht befindet. Mit dieser Maßnahme kann das Katalysatorsystem im Reaktor in seiner Aktivität dem Reaktionsverlauf entsprechend angepasst werden.

Sehr unterschiedliche Arten der Aktivitätssteigerung sind im Stand der Technik beschrieben, beispielsweise:

In der DE-A-22 38 067 wird der Einsatz von zwei Katalysatorzonen unterschiedlicher Aktivität beschrieben. Die Aktivmassen unterscheiden sich im Anteil der Kaliumionen.

DE-A-198 23 275 beschreibt ein zweischichtiges Katalysatorsystem. Die Aktivitätsstrukturierung erfolgt über die Aktivmassenmenge auf dem Träger und über die Menge an zugesetzten Dotierungen in Form von Alkalimetallverbindungen in der Aktivmasse (siehe auch WO 03/70680).

In der EP-A 1 063 222 wird beim Einsatz von drei oder mehrlagigen Katalysatorsystemen die Aktivität der einzelnen Zonen durch die Phosphormenge der Aktivmasse, die Menge der Aktivmasse auf dem Trägerring, die Menge der Alkalidotierung der Aktivmasse und der Füllhöhe der einzelnen Katalysatorlagen im Reaktionsrohr verändert.

WO 98/17608 beschreibt eine Aktivitätsstrukturierung mit Hilfe unterschiedlicher Porosität der verschiedenen Katalysatorschichten. Die Porosität wird durch das freie Volumen zwischen den beschichteten Formkörpern der Schüttung im Reaktionsrohr definiert.

In den einzelnen Katalysatorschichten ist Titandioxid in der Anatasmodifikation der Hauptbestandteil der Aktivmasse der Phthalsäureanhydridkatalysatoren und dient zur Trägerung der katalytisch aktiven und selektiven Vanadiumpentoxid Komponenten neben anderen Metalloxiden.

DE-A 21 06 796 beschreibt die Herstellung von Trägerkatalysatoren zur Oxidation von o-Xylol zu Phthalsäureanhydrid, wobei das Titandioxid eine BET-Oberfläche von 15 bis 100 $m^2/g$, vorzugsweise 25 bis 50 $m^2/g$ aufweist. Es wird offenbart, dass Mischungen aus Anatas der BET-Oberfläche von 7 bis 11 $m^2/g$ und Titandioxid-Hydrat der BET-Oberfläche > 100 $m^2/g$ besonders geeignet sind, wobei die Komponenten alleine nicht geeignet wären.

In der EP-A 744 214 werden Mischung von Titandioxid mit einer BET-Oberfläche von 5 bis 11$m^2/g$ und Titandioxidhydrat mit einer BET-Oberfläche von mehr als 100 $m^2/g$ in einem Mischungsverhältnis von 1:3 bis 3:1 beschrieben.

Eine Mischung von Titandioxiden mit einer BET-Oberfläche von 7 bis 11 $m^2/g$ mit Titandioxid-Hydrat mit einer BET-Oberfläche von >100 $m^2/g$ ist ferner in der DE-A 196 33 757 beschrieben. Beide Komponenten können im Verhältnis, bezogen auf ein Gramm $TiO_2$, von 1:9 bis 9:1 enthalten sein.
Weiter ist eine Mischung von Titandioxid mit Titandioxidhydrat im Mengenverhältnis 3:1 in der DE-A 22 38 067 beschrieben.

Ein Problem dieser Mischungen von Titandioxid mit Titandioxidhydraten stellt die Abnahme der BET-Oberflächen dieser Mischungen über die Lebensdauer dar.

In der EP-A 522 871 wird ein Zusammenhang zwischen der BET-Oberfläche des Titandioxids und der Katalysatoraktivität beschrieben. Gemäß dieser Schrift ist die Katalysatoraktivität bei einem Einsatz von Titandioxid mit BET-Oberflächen von unter 10 $m^2/g$ gering. Bei einem Einsatz von Titandioxid mit einer BET-Oberfläche größer als 60 $m^2/g$ ist die Lebensdauer des Katalysators reduziert und die Phthalsäureanhydridausbeute sinkt stark ab. Bevorzugt sind BET-Oberflächen von 15 bis 40 $m^2/g$.

Bei mehrlagigen Katalysatorsystemen wirkt sich die Abnahme der Aktivität der ersten Katalysatorschicht im Bezug auf die Lebensdauer des Katalysators negativ aus. Mit zunehmender Alterung geht der Umsatz im Bereich der ersten hochselektiven Schicht zurück. Die Hauptreaktionszone wandert im Laufe der Katalysatorlebenszeit immer tiefer in das Katalysatorbett, d.h. der o-Xylol- oder Naphthalinfeed wird immer häufiger erst in den nachfolgenden weniger selektiven Schichten umgesetzt. Die Folge sind verringerte Phthalsäureanhydridausbeuten und eine erhöhte Konzentration an Nebenprodukten oder nicht umgesetzten Edukten. Um das Wandern der Hauptreaktionszone in die nachfolgenden Schichten zu vermeiden, kann die Salzbadtemperatur stetig angehoben werden. Mit zunehmender Lebensdauer der Katalysatoren führt allerdings auch diese Maßnahme zur Verringerung der Phthalsäureanhydridausbeute.

Die Ausbeute an Phthalsäureanhydrid ist ferner umso geringer, je höher die Beladung der Luft mit dem zu oxidierenden Kohlenwasserstoff ist, da eine hohe Beladung das Wandern der Hauptreaktionszone tiefer in das Katalysatorbett verstärkt. Für eine wirtschaftliche Herstellung sind aber hohe Beladungen von 80 bis 120 g/$Nm^3$ erwünscht.

Eine hohe Beladung führt demnach zu einer schnelleren Schädigung der Katalysatoren und somit zu kürzeren Standzeiten.

Der vorliegenden Erfindung lag daher die Aufgabe zugrunde, ein Verfahren zur Herstellung von Phthalsäureanhydrid zur Verfügung zu stellen, das trotz hoher Beladung Phthalsäureanhydrid in hoher Ausbeute und guter Qualität, d.h. insbesondere mit geringem Phthalidanteil, liefert. Weiterhin soll die Standzeit der Katalysatoren verbessert werden.

Überraschenderweise wurde nun gefunden, dass diese Aufgabe mittels eines Katalysatorsystems gelöst werden konnte, das mindestens drei im Reaktionsrohr übereinander angeordnete Katalysatorschichten aufweist, deren Katalysatoraktivität von Schicht zu Schicht ansteigt und deren Aktivmasse neben Metalloxiden 70 bis 99 Gew.-% Titandioxid in der Anatasmodifikation enthält, wobei

(i) der am geringsten aktive Katalysator auf Trägermaterial 7 bis 10 Gew.-%. bezogen auf den gesamten Katalysator, Aktivmasse aufweist, enthaltend 6 bis 11 Gew.-% $V_2O_5$, 0 bis 3 Gew.-% $Sb_2O_3$, 0,1 bis 1 Gew.-% Alkali (ber. als Alkalimetall) und als Rest $TiO_2$ in Anatasform mit einer BET-Oberfläche von 5 bis 30 $m^2/g$,

(ii) der nächst aktivere Katalysator auf Trägermaterial 7 bis 12 Gew.-%, bezogen auf den gesamten Katalysator, Aktivmasse aufweist, enthaltend 5 bis 13 Gew.-% $V_2O_5$, 0 bis 3 Gew.-% $Sb_2O_3$, 0 bis 0,4 Gew.-% P, 0 bis 0,4 Gew.-% Alkali (ber. als Alkalimetall) und als Rest $TiO_2$ in Anatasform mit einer BET-Oberfläche von 10 bis 40 $m^2/g$,

(iii) der aktivste Katalysator auf Trägermaterial 8 bis 12 Gew.-%, bezogen auf den gesamten Katalysator, Aktivmasse aufweist, enthaltend 5 bis 30 Gew.-% $V_2O_5$, 0 bis 3 Gew.-% $Sb_2O_3$, 0,05 bis 0,4 Gew.-% P, 0 bis 0,3 Gew.-% Alkali (ber. als Alkalimetall) und als Rest $TiO_2$ in Anatasform mit einer BET-Oberfläche von 15 bis 50 $m^2/g$,

mit der Maßgabe, dass die BET-Oberfläche des Titandioxids in der obersten Schicht (i) kleiner ist als die BET-Oberfläche des Titandioxids in der oder den mittleren Schichten (ii) und die BET-Oberfläche des Titandioxids in der untersten Schicht (iii) größer ist als die BET-Oberfläche des Titandioxids in der oder den mittleren Schichten.

Weiterhin wurde gefunden, dass sich Phthalsäureanhydrid vorteilhaft unter Verwendung des erfindungsgemäßen Katalysatorsystems herstellen lässt.

Bevorzugt besteht das Katalysatorsystem aus drei bis fünf Schichten, insbesondere aus vier Schichten. Im Falle eines dreilagigen Katalysatorsystems gilt für die BET-Oberfläche der eingesetzten Tiandioxide in Anatasmodifi-kation:

$$BET_{Zone(i)} < BET_{Zone(ii)} < BET_{Zone(iii)}$$

im Falle eines vierlagigen Katalysatorsystems:

$$BET_{Zone(i)} < BET_{Zone(iia)} \leq BET_{Zone(iib)} < BET_{Zone(iii)}$$

und im Falle eines fünflagigen Katalysatorsystems:

$$BET_{Zone(i)} < BET_{Zone(iia)} \leq BET_{Zone(iib)} \leq BET_{Zone(iic)} < BET_{Zone(iii)}$$

Bevorzugt weist das Titandioxid

(i) in der obersten Schicht eine BET-Oberfläche von 7 bis 25 $m^2/g$ auf,
(ii) in der oder den mittleren Schichten eine BET-Oberfläche von 10 bis 35 $m^2/g$ auf, und
(iii) in der untersten Schicht eine BET-Oberfläche von 15 bis 45 $m^2/g$ auf.

In einem vierlagigen Katalysatorsystem weist das Titandioxid der oberen Mittelschicht (iia) beispielsweise eine BET-Oberfläche von 10 bis 35 $m^2/g$, insbesondere 10 bis 30 $m^2/g$, und das Titandioxid der untere Mittelschicht (iib) eine BET-Oberfläche von 15 bis 40 $m^2/g$, insbesondere 15 bis 35 $m^2/g$, auf.

In einem fünflagigen Katalysatorsystem weist das Titandioxid der oberen Mittelschicht (iia) beispielsweise eine BET-Oberfläche von 10 bis 35 $m^2/g$, insbesondere 10 bis 30 $m^2/g$, der mittleren Mittelschicht (iib) eine BET-Oberfläche von 10 bis 40 m$^2$/g, insbesondere 10 bis 35 m$^2$/g, und der unteren Mittelschicht (iic) eine BET-Oberfläche von 15 bis 40 m$^2$/g, insbesondere 15 bis 38 m$^2$/g, auf.

Bevorzugt besteht das eingesetzte Titandioxid in mindestens einer Katalysatorschicht aus einem Gemisch von Titandioxiden unterschiedlicher BET-Oberflächen. Dieses Gemisch aus Titandioxid Typen beinhaltet beispielsweise ein niederoberflächiges Titandioxid mit einer BET-Oberfläche von vorteilhaft 5 bis 15 m$^2$/g, insbesondere 5 bis 10 m$^2$/g, und ein höheroberflächiges Titandioxid mit einer BET-Oberfläche von vorteilhaft 10 bis 70 m$^2$/g, insbesondere 15 bis 50 m$^2$/g. Insbesondere besteht das eingesetzte Titandioxid aus den zwei genannten Titandioxid Typen.

Gegenüber den im Stand der Technik beschriebenen Titanoxidhydraten und deren Mischung mit niederoberflächigem TiO$_2$ hat das erfindungsgemäß eingesetzte Gemisch den Vorteil, dass sich die BET-Oberfläche über die Lebensdauer des erfindungsgemäßen Katalysators nicht verändert. Somit wird eine hohe Stabilität der Aktivität, d.h. eine längere Lebensdauer des Katalysators, gewährleistet.

Das eingesetzte Titandioxid besteht vorteilhaft aus einem Gemisch eines Titandioxids mit einer BET-Oberfläche von 5 bis 15 m$^2$/g und einem Titandioxid mit einer BET-Oberfläche von 15 bis 50 m$^2$/g in einem Verhältnis von

(i) 1:1,5 bis 1:3 in der obersten Schicht
(iia) 1:2 bis 1:4 in der oberen mittleren Schicht
(iib) 1:2,5 bis 1:4 in der unteren mittleren Schicht und
(iii) 1:3 bis 1:5 in der untersten Schicht.

Die Schüttungslänge der obersten Katalysatorschicht (i) beträgt vorteilhaft 80 bis 160 cm, die der oberen mittleren Katalysatorschicht (iia) 20 bis 60 cm, die der unteren mittleren Katalysatorschicht (iib) 30 bis 100 cm und die der untersten Katalysatorschicht (iii) 40 bis 90 cm.

Als Katalysatoren sind oxidische Trägerkatalysatoren geeignet. Zur Herstellung von Phthalsäureanhydrid durch Gasphasenoxidation von o-Xylol oder Naphthalin oder Gemischen davon verwendet man in der Regel kugelförmige, ringförmige oder schalenförmige Träger aus einem Silikat, Siliciumcarbid, Porzellan, Aluminiumoxid, Magnesiumoxid, Zinndioxid, Rutil, Aluminiumsilikat, Magnesiumsilicat (Steatit), Zirkoniumsilicat oder Cersilicat oder Mischungen davon. Besonders bewährt haben sich sogenannte Schalenkatalysatoren, bei denen die katalytisch aktive Masse schalenförmig auf den Träger aufgebracht ist. Als katalytisch aktiver Bestandteil dient vorzugsweise Vanadiumpentoxid. Weiter können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Derartige Promotoren sind beispielsweise die Alkalimetalloxide, Thallium(I)oxid, Aluminiumoxid, Zirkoniumoxid, Eisenoxid, Nickeloxid, Cobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid. Die Alkalimetalloxide wirken beispielsweise als die Aktivität vermindernde und die Selektivität erhöhende Promotoren. Weiterhin können der katalytisch aktiven Masse organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat, Vinylacetat/Ethylen sowie Hydroxyethylcellulose zugesetzt werden, wobei Bindermengen von 3 bis 20 Gew.-%, bezogen auf den Feststoffgehalt der Lösung der Aktivmassenbestandteile, eingesetzt wurden (EP-A 744 214). Bevorzugt werden organische Binder wie in der DE-A 198 24 532 beschrieben verwendet. Wird die katalytisch aktive Masse ohne organische Bindemittel auf den Träger aufgetragen, so sind Beschichtungstemperaturen über 150°C von Vorteil. Bei Zusatz der oben angegebenen Bindemittel liegen die brauchbaren Beschichtungstemperaturen je nach verwendetem Bindemittel zwischen 50 und 450°C (DE-A 21 06 796). Die aufgetragenen Bindemittel brennen nach dem Einfüllen des Katalysators und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

Das dem Katalysator zugeführte Reaktionsgas (Ausgangsgasgemisch) wird im allgemeinen durch Vermischen von einem molekularen Sauerstoff enthaltenden Gas, das außer Sauerstoff noch geeignete Reaktionsmoderatoren, wie Stickstoff und/oder Verdünnungsmittel, wie Dampf und/oder Kohlendioxid, enthalten kann, mit dem zu oxidierenden, aromatischen Kohlenwasserstoff erzeugt. Das molekularen Sauerstoff enthaltende Gas kann im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten. Zur Erzeugung des Reaktionsgases wird das molekularen Sauerstoff enthaltende Gas im allgemeinen mit 30 g bis 150 g je Nm$^3$ Gas, insbesondere mit 60 bis 120 g je Nm$^3$, des zu oxidierenden, aromatischen Kohlenwasserstoffs beschickt.

In mehrlagigen Katalysatorsystemen wird im Allgemeinen der weniger aktive Katalysator so im Festbett angeordnet, dass das Reaktionsgas zuerst mit diesem Katalysator und erst im Anschluß daran mit dem aktiveren Katalysator in der zweiten Schicht in Kontakt kommt. Anschließend kommt das Reaktionsgas mit den noch aktiveren Katalysatorschichten in Kontakt. Die unterschiedlich aktiven Katalysatoren können auf die gleiche oder auf unterschiedliche Temperaturen thermostatisiert werden.

Über die so bereitete Katalysatorschüttung wird das Reaktionsgas bei Temperaturen von im allgemeinen 300 bis 450°C, vorzugsweise 320 bis 420°C und besonders bevorzugt von 340 bis 400°C geleitet. Es wird vorteilhaft ein Überdruck von im allgemeinen 0,1 bis 2,5 bar, vorzugsweise von 0,3 bis 1,5 bar, verwendet. Die Raumgeschwindigkeit liegt im allgemeinen bei 750 bis 5000 $h^{-1}$.

Die Hot-Spot-Temperatur der obersten Schicht beträgt vorzugsweise 400 bis 470°C, insbesondere liegt die maximal Temperatur unter 450°C. In der oder den Mittelschichten eines mehrlagigen Katalysatorsystems ist die Hot-Spot-Temperatur vorteilhaft kleiner als 420°C, insbesondere kleiner 410°C.

In einer bevorzugten Ausführungsform eines dreilagigen Katalysatorsystems weisen die Katalysatoren beispielsweise folgende Zusammensetzung auf:

- für die erste, oberste Schicht (Schicht (i)):

  7 bis 10 Gew.-% Aktivmasse bezogen auf den gesamten Katalysator, wobei diese Aktivmasse:
  6 bis 11 Gew.-% Vanadiumpentoxid
  0 bis 3 Gew.-% Antimontrioxid
  0,1 bis 1 Gew.-% eines Alkali (ber. als Alkalimetall), insbesondere Cäsiumoxid enthält und als Rest zu 100 Gew.-% Titandioxid in Anatasmodifikation mit einer BET-Oberfläche von 5 bis 30 $m^2$/g

- für die zweite, mittlere Schicht (Schicht (ii)):

  7 bis 12 Gew.-% Aktivmasse bezogen auf den gesamten Katalysator, wobei diese Aktivmasse:
  5 bis 13 Gew.-% Vanadiumpentoxid
  0 bis 3 Gew.-% Antimontrioxid
  0 bis 0,4 Gew.-% eines Alkali (ber. als Alkalimetall), insbesondere Cäsiumoxid
  0 bis 0,4 Gew.-% Phosphorpentoxid (berechnet als P)
  enthält und als Rest zu 100 Gew.-% Titandioxid in Anatasmodifikation mit einer BET-Oberfläche von 10 bis 40 $m^2$/g

- für die dritte, unterste Schicht (Schicht (iii)):

  8 bis 12 Gew.-% Aktivmasse bezogen auf den gesamten Katalysator, wobei diese Aktivmasse:
  5 bis 30 Gew.-% Vanadiumpentoxid
  0 bis 3 Gew.-% Antimontrioxid
  0 bis 0,3 Gew.-% eines Alkali (ber. als Alkalimetall), insbesondere Cäsiumoxid
  0,05 bis 0,4 Gew.-% Phosphorpentoxid (berechnet als P)
  enthält und als Rest zu 100 Gew.-% Titandioxid, insbesondere in Anatasmodifikation mit einer BET-Oberfläche von 15 bis 50 $m^2$/g.

In einer bevorzugten Ausführungsform eines vierlagigen Katalysatorsystems weisen die Katalysatoren beispielsweise folgende Zusammensetzung auf:

- für die erste Schicht (Schicht (i)):

  7 bis 10 Gew.-% Aktivmasse bezogen auf den gesamten Katalysator, wobei diese Aktivmasse:
  6 bis 11 Gew.-% Vanadiumpentoxid
  0 bis 3 Gew.-% Antimontrioxid
  0,1 bis 1 Gew.% eines Alkali (ber. als Alkalimetall), insbesondere Cäsiumoxid
  enthält und als Rest zu 100 Gew.% Titandioxid in Anatasmodifikation mit einer BET-Oberfläche von 5 bis 30 $m^2$/g

- für die zweite Schicht (Schicht (iia)):

  7 bis 12 Gew.% Aktivmasse bezogen auf den gesamten Katalysator, wobei diese Aktivmasse:
  4 bis 15 Gew.-% Vanadiumpentoxid
  0 bis 3 Gew.-% Antimontrioxid
  0,1 bis 1 Gew.-% eines Alkali (ber. als Alkalimetall), insbesondere Cäsiumoxid
  0 bis 0,4 Gew.-% Phosphorpentoxid (berechnet als P)
  enthält und als Rest zu 100 Gew.-% Titandioxid in Anatasmodifikation mit einer BET-Oberfläche von 10 bis 35 $m^2$/g

- für die dritte Schicht (Schicht (iib)):

7 bis 12 Gew.-% Aktivmasse bezogen auf den gesamten Katalysator, wobei diese Aktivmasse:
5 bis 15 Gew.-% Vanadiumpentoxid
0 bis 3 Gew.-% Antimontrioxid
0 bis 0,4 Gew.-% eines Alkali (ber. als Alkalimetall), insbesondere Cäsiumoxid
0 bis 0,4 Gew.-% Phosphorpentoxid (berechnet als P)
enthält und als Rest zu 100 Gew.-% Titandioxid in Anatasmodifikation mit einer BET-Oberfläche von 15 bis 40 $m^2$/g

- für die vierte Schicht (Schicht (iii)):

8 bis 12 Gew.-% Aktivmasse bezogen auf den gesamten Katalysator, wobei diese Aktivmasse:
5 bis 30 Gew.-% Vanadiumpentoxid
0 bis 3 Gew.-% Antimontrioxid
0,05 bis 0,4 Gew.-% Phosphorpentoxid (berechnet als P)
enthält und als Rest zu 100 Gew.-% Titandioxid in Anatasmodifikation mit einer BET-Oberfläche von 15 bis 50 $m^2$/g.

Gewünschtenfalls kann man für die Phthalsäureanhydridherstellung noch einen nachgeschalteten Finishing-Reaktor vorsehen, wie er beispielsweise in der

DE-A 198 07 018 oder DE-A 20 05 969 beschrieben ist. Als Katalysator verwendet man dabei im Vergleich zum Katalysator der letzten Schicht vorzugsweise einen noch aktiveren Katalysator.

Durch das erfindungsgemäße Katalysatorsystem konnten die Standzeiten durch eine gleichmäßigere Reaktionswärmeverteilung über die Katalysatorschüttung erhöht werden. Somit sinkt die maximale Hot-spot-Temperatur und die PhthalsäureanhydridAusbeute kann bei geringen Nebenproduktkonzentrationen gesteigert werden.

Phthalsäureanhydrid lässt sich erfindungsgemäß auch bei hohen Beladungen, beispielsweise bei 80 bis 120 g/Nm³, mit o-Xylol und/oder Naphthalin und bei hohen Raumgeschwindigkeiten mit hoher Ausbeute und geringen Konzentrationen an Nebenprodukt, insbesondere Phthalid, herstellen. Unter den Bedingungen des erfindungsgemäßen Verfahrens ist die Phthalid-Konzentration nicht höher als 0,05 Gew.-%, bezogen auf Phthalsäureanhydrid.

Beispiele:

Katalysator 1: 4 Lagen

Oberschicht (i)

29,3 g Anatas (BET-OF 9 $m^2$/g), 69,8 g Anatas (BET-OF 20 $m^2$/g), 7,8 $V_2O_5$, 1,9 g $Sb_2O_3$, 0,49 g $Cs_2CO_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, Außendurchmesser (AD) x Länge (L) x Innendurchmesser (ID)) durch Aufsprühen. Das Gewicht der aufgetragenen Aktivmassenschale betrug 8 % des Gesamtgewichtes des fertigen Katalysators.
Die auf diese Weise aufgebrachte katalytisch aktive Masse enthielt nach Kalzination bei 400°C für 4h 7,1 Gew.-% $V_2O_5$, 1,8 Gew.-% $Sb_2O_3$, 0,36 Gew.-% Cs.
Die BET-Oberfläche der $TiO_2$ Mischung betrug 16,7 $m^2$/g.

Obere Mittelschicht (iia)

24,6 g Anatas (BET-OF 9 $m^2$/g), 74,5 g Anatas (BET-OF 27 $m^2$/g), 7,8 $V_2O_5$, 2,6 g $Sb_2O_3$, 0,35 g $Cs_2CO_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.
Die auf diese Weise aufgebrachte katalytisch aktive Masse enthielt nach Kalzination bei 400°C für 4h 7,1 Gew.-% $V_2O_5$, 2,4 Gew.-% $Sb_2O_3$, 0,26 Gew.-% Cs.
Die BET-Oberfläche der $TiO_2$ Mischung betrug 22,5 $m^2$/g.

Untere Mittelschicht (iib)

24,8 g Anatas (BET-OF 9 m$^2$/g), 74,5 g Anatas (BET-OF 27 m$^2$/g), 7,8 V$_2$O$_5$, 2,6 g Sb$_2$O$_3$, 0,13 g Cs$_2$CO$_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.
Die auf diese Weise aufgebrachte katalytisch aktive Masse enthielt nach Kalzination bei 400°C für 4h 7,1 Gew.-% V$_2$O$_5$, 2,4 Gew.-% Sb$_2$O$_3$, 0,10 Gew.-% Cs.
Die BET-Oberfläche der TiO$_2$ Mischung betrug 22,5 m$^2$/g.

Unterschicht (iii)

17,2 g Anatas (BET-OF 9 m$^2$/g), 69,1 g Anatas (BET-OF 27 m$^2$/g), 21,9 V$_2$O$_5$, 1,5 g NH$_4$H$_2$PO$_4$ wurden in 550 ml entionisiertem Wasser suspendiert und 15 Stunden lang gerührt. Dieser Suspension wurden anschließend 55 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichtes des fertigen Katalysators.
Die auf diese Weise aufgebrachte katalytisch aktive Masse enthielt nach Kalzination bei 400°C für 4h 20,0 Gew.-% V$_2$O$_5$, 0,38 Gew.-% P.
Die BET-Oberfläche der TiO$_2$ Mischung betrug 23,4 m$^2$/g.

Katalysator 2: 4 Lagen

Oberschicht (i)

29,3 g Anatas (BET-OF 9 m$^2$/g), 69,8 g Anatas (BET-OF 20 m$^2$/g), 7,8 V$_2$O$_5$, 1,9 g Sb$_2$O$_3$, 0,49 g Cs$_2$CO$_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.
Die auf diese Weise aufgebrachte katalytisch aktive Masse enthielt nach Kalzination bei 400°C für 4h 7,1 Gew.-% V$_2$O$_5$, 1,8 Gew.-% Sb$_2$O$_3$, 0,36 Gew.-% Cs.
Die BET-Oberfläche der TiO$_2$ Mischung betrug 16,7 m$^2$/g.

Obere Mittelschicht (iia)

24,6 g Anatas (BET-OF 9 m$^2$/g), 74,5 g Anatas (BET-OF 20 m$^2$/g), 7,8 V$_2$O$_5$, 2,6 g Sb$_2$O$_3$, 0,35 g Cs$_2$CO$_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.
Die auf diese Weise aufgebrachte katalytisch aktive Masse enthielt nach Kalzination bei 400°C für 4h 7,1 Gew.-% V$_2$O$_5$, 2,4 Gew.-% Sb$_2$O$_3$, 0,26 Gew.-% Cs.
Die BET-Oberfläche der TiO$_2$ Mischung betrug 17,3 m$^2$/g.

Untere Mittelschicht (iib)

24,8 g Anatas (BET-OF 9 m$^2$/g), 74,5 g Anatas (BET-OF 20 m$^2$/g), 7,8 V$_2$O$_5$, 2,6 g Sb$_2$O$_3$, 0,13 g Cs$_2$CO$_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD × L x ID) durch Aufsprühen. Das Gewicht der aufgetragenen Aktivmassenschale betrug 8 % des Gesamtgewichtes des fertigen Katalysators.

Die auf diese Weise aufgebrachte katalytisch aktive Masse enthielt nach Kalzination bei 400°C für 4h 7,1 Gew.-% $V_2O_5$, 2,4 Gew.-% $Sb_2O_3$, 0,10 Gew.-% Cs.

Die BET-Oberfläche der $TiO_2$ Mischung betrug 17,3 $m^2$/g.

Unterschicht (iii)

17,2 g Anatas (BET-OF 9 $m^2$/g), 69,1 g Anatas (BET-OF 27 $m^2$/g), 21,9 $V_2O_5$, 1,5 g $NH_4H_2PO_4$ wurden in 550 ml entionisiertem Wasser suspendiert und 15 Stunden lang gerührt. Dieser Suspension wurden anschließend 55 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators. Die auf diese Weise aufgebrachte katalytisch aktive Masse enthielt nach Kalzination bei 400°C für 4 h 20,0 Gew.-% $V_2O_5$, 0,38 Gew.-% P.

Die SET-Oberfläche der $TiO_2$ Mischung betrug 23,4 $m^2$/g.

Katalysator 3: 4 Lagen (Vergleichsbeispiel)

Oberschicht (i)

99,5 g Anatas (BET-OF 20 $m^2$/g), 7,8 $V_2O_5$, 1,9 g $Sb_2O_3$, 0,49 g $Cs_2CO_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.

Die BET-Oberfläche des $TiO_2$ betrug 20,1 $m^2$/g.

Obere Mittelschicht (iia)

99,3 g Anatas (BET-OF 20 $m^2$/g), 7,8 $V_2O_5$, 2,6 g $Sb_2O_3$, 0,35 g $Cs_2CO_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.

Die BET-Oberfläche des $TiO_2$ betrug 20,0 $m^2$/g.

Untere Mittelschicht (iib)

99,0 g Anatas (BET-OF 20 $m^2$/g), 7,8 $V_2O_5$, 2,6 g $Sb_2O_3$, 0,13 g $Cs_2CO_3$ wurden in 550 ml entionisiertem Wasser suspendiert und 15 Stunden lang gerührt. Dieser Suspension wurden anschließend 50 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.

Die BET-Oberfläche des $TiO_2$ betrug 20,3 $m^2$/g.

Unterschicht (iii)

86,5 g Anatas (BET-OF 20 $m^2$/g), 21,9 $V_2O_5$, 1,5 g $NH_4H_2PO_4$ wurden in 550 ml entionisiertem Wasser suspendiert und 15 Stunden lang gerührt. Dieser Suspension wurden anschließend 55 g einer wässerigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8 % des Gesamtgewichtes des fertigen Katalysators.

Die BET-Oberfläche des $TiO_2$ betrug 20,2 $m^2$/g.

Katalytische Tests:

Die Tests erfolgten in einem salzbadgekühltem Reaktor mit einer Länge von 3,85 m und einem Innendurchmesser von 25 mm. Zur Aufnahme eines Temperaturprofils war der Reaktor mit einem über die gesamte Reaktorlänge beweglichen Thermoelement ausgestattet. Das Element wurde in einer Hülse mit einem Außendurchmesser von 2 mm gehalten. Durch das Rohr wurden stündlich 4 Nm$^3$-Luft mit o-Xylol (mindestens 98,5 % Reinheit) von 0 bis 100 g/Nm$^3$ geleitet. Dabei wurden die unten zusammengefassten Ergebnisse erhalten ("PSA-Aubeute" bedeutet das erhaltene PSA in Gewichtprozent, bezogen auf 100%iges o-Xylol).

Tabelle 1: Ergebnisse der katalytischen Tests

|  | Katalysator 1 | Katalysator 2 | Katalysator 3 nicht erfindungsgemäß |
|---|---|---|---|
| Schüttungslängen [cm] | 130, 50, 80, 60 | 130, 50, 70, 70 | 130, 50, 70, 70 |
| Beladung o-Xylol [g/Nm$^3$] | 100 | 100 | 80 |
| Laufzeit [d] | 43 | 40 | 20 |
| SBT [°C] | 354 | 360 | 347 |
| HST-OS [°C] | 440 | 440 | 452 |
| PHD [Gew.-%] | 0,02 | 0,01 | 0,03 |
| PSA-Ausbeute [Gew.-%] | 113,5 | 113,7 | 111,3 |

Folgende Abkürzungen wurden verwendet:

HST-OS     Hot Spot-Temperatur in der Oberschicht
SBT         Salzbadtemperatur
PHD        Phthalid
PSA        Phthalsäureanhydrid

**Patentansprüche**

1. Katalysatorsystem zur Herstellung von Phthalsäureanhydrid, das mindestens drei im Reaktionsrohr übereinander angeordnete Katalysatorschichten aufweist, deren Katalysatoraktivität von Schicht zu Schicht ansteigt und deren Aktivmasse 70 bis 99 Gew.-% Titandioxid in der Anatasmodifikation enthält, wobei

(i) der am geringsten aktive Katalysator auf Trägermaterial 7 bis 10 Gew.-%, bezogen auf den gesamten Katalysator, Aktivmasse aufweist, enthaltend 6 bis 11 Gew.-% $V_2O_5$, 0 bis 3 Gew.-% $Sb_2O_3$, 0,1 bis 1 Gew.-% Alkali (ber. als Alkalimetall) und als Rest $TiO_2$ in Anatasform mit einer BET-Oberfläche von 5 bis 30 m$^2$/g,
(ii) der nächst aktivere Katalysator auf Trägermaterial 7 bis 12 Gew.-%, bezogen auf den gesamten Katalysator, Aktivmasse aufweist, enthaltend 5 bis 13 Gew.-% $V_2O_5$, 0 bis 3 Gew.-% $Sb_2O_3$, 0 bis 0,4 Gew.-% P, 0 bis 0,4 Gew.-% Alkali (ber. als Alkalimetall) und als Rest $TiO_2$ in Anatasform mit einer BET-Oberfläche von 10 bis 40 m$^2$/g,
(iii) der aktivste Katalysator auf Trägermaterial 8 bis 12 Gew.-%, bezogen auf den gesamten Katalysator, Aktivmasse aufweist, enthaltend 5 bis 30 Gew.-% $V_2O_5$, 0 bis 3 Gew.-% $Sb_2O_3$, 0,05 bis 0,4 Gew.-% P, 0 bis 0,3 Gew.-% Alkali (ber. als Alkalimetall) und als Rest $TiO_2$ in Anatasform mit einer BET-Oberfläche von 15 bis 50 m$^2$/g,

mit der Maßgabe, dass die BET-Oberfläche des Titandioxids in der obersten Schicht (i) kleiner ist als die BET-Oberfläche des Titandioxids in der oder den mittleren Schichten (ii) und die BET-Oberfläche des Titandioxids in der untersten Schicht (iii) größer ist als die BET-Oberfläche des Titandioxids in der oder den mittleren Schichten.

2. Katalysatorsystem nach Anspruch 1, in dem zwei mittlere Schichten (ii) vorliegen, deren Titandioxid in der oberen Mittelschicht eine BET-Oberfläche von 10 bis 35 m$^2$/g und in der unteren Mittelschicht eine BET-Oberfläche von 15 bis 40 m$^2$/g aufweist, mit der Maßgabe, dass die BET-Oberfläche des Titandioxids in der oberen Mittelschicht kleiner oder gleich der BET-Oberfläche der unteren Mittelschicht ist.

3. Katalysatorsystem nach Anspruch 1 oder 2, wobei das Titandioxid in mindestens einer Katalysatorschicht aus einem Gemisch von Titandioxiden unterschiedlicher BET-Oberfläche besteht.

4. Katalysatorsystem nach Anspruch 3, wobei das Gemisch aus einem Titandioxid mit einer BET-Oberfläche von 5 bis 15 m$^2$/g und aus einem Titandioxid mit einer BET-Oberfläche von 15 bis 50 m$^2$/g in einem Verhältnis

(i) von 1:1,5 bis 1:3 in der obersten Schicht,
(iia) von 1:2 bis 1:4 in der oberen mittleren Schicht,
(iib) von 1:2,5 bis 1:4 in der unteren mittleren Schicht und
(iii) von 1:3 bis 1:5 in der untersten Schicht

besteht.

**5.** Katalysatorsystem nach den Ansprüchen 1 bis 4, wobei die Schüttungslänge

(i) der obersten Katalysatorschicht 80 bis 160 cm,
(iia) der oberen mittleren Katalysatorschicht 20 bis 60 cm,
(iib) der unteren mittleren Katalysatorschicht 30 bis 100 cm und
(iii) der untersten Katalysatorschicht 40 bis 90 cm beträgt.

**6.** Katalysatorsystem nach den Ansprüchen 1 bis 5, das vier übereinander angeordnete Schichten aufweist, wobei

(i) der am geringsten aktive Katalysator auf Trägermaterial 7 bis 10 Gew.-%. bezogen auf den gesamten Katalysator, Aktivmasse aufweist, enthaltend 6 bis 11 Gew.-% $V_2O_5$, 0 bis 3 Gew.-% $Sb_2O_3$, 0,1 bis 1 Gew.-% Alkali (ber. als Alkalimetall) und als Rest $TiO_2$ in Anatasform mit einer BET-Oberfläche von 5 bis 30 $m^2/g$,
(iia) der nächst aktivere Katalysator auf Trägermaterial 7 bis 12 Gew.-%, bezogen auf den gesamten Katalysator, Aktivmasse aufweist, enthaltend 4 bis 15 Gew.-% $V_2O_5$, 0 bis 3 Gew.-% $Sb_2O_3$, 0,1 bis 1 Gew.-% Alkali (ber. als Alkalimetall), 0 bis 0,4 Gew.-% P und als Rest $TiO_2$ in Anatasform mit einer BET-Oberfläche von 10 bis 35 $m^2/g$,
(iib) der nächst aktivere Katalysator auf Trägermaterial 7 bis 12 Gew.-%, bezogen auf den gesamten Katalysator, Aktivmasse aufweist, enthaltend 5 bis 15 Gew.-% $V_2O_5$, 0 bis 3 Gew.-% $Sb_2O_3$, 0 bis 0,4 Gew.-% Alkali (ber. als Alkalimetall), 0 bis 0,4 Gew.-% P und als Rest $TiO_2$ in Anatasform mit einer BET-Oberfläche von 15 bis 40 $m^2/g$,
(iii) der aktivste Katalysator auf Trägermaterial 8 bis 12 Gew.-%, bezogen auf den gesamten Katalysator, Aktivmasse aufweist, enthaltend 5 bis 30 Gew.-% $V_2O_5$, 0 bis 3 Gew.-% $Sb_2O_3$, 0,05 bis 0,4 Gew.-% P und als Rest $TiO_2$ in Anatasform mit einer BET-Oberfläche von 15 bis 50 $m^2/g$.

**7.** Verfahren zur Herstellung von Phthalsäureanhydrid durch Gasphasenoxidation von Xylol, Naphthalin oder Gemischen davon in einem Rohrbündelreaktor, **dadurch gekennzeichnet, dass** die Ausgangsstoffe über ein Katalysatorsystem nach den Ansprüchen 1 bis 6 geleitet werden.

**Claims**

**1.** A catalyst system for preparing phthalic anhydride, which comprises at least three catalyst layers which are arranged above one another in the reaction tube, whose catalyst activity increases from layer to layer and whose active composition comprises from 70 to 99% by weight of titanium dioxide in the anatase modification, wherein

(i) the least active catalyst comprises from 7 to 10% by weight, based on the total catalyst, of active composition comprising from 6 to 11 % by weight of $V_2O_5$, from 0 to 3% by weight of $Sb_2O_3$, from 0.1 to 1 % by weight of alkali (calculated as alkali metal) and $TiO_2$ in anatase form having a BET surface area of from 5 to 30 $m^2/g$ as balance on support material,
(ii) the next more active catalyst comprises from 7 to 12% by weight, based on the total catalyst, of active composition comprising from 5 to 13% by weight of $V_2O_5$, from 0 to 3% by weight of $Sb_2O_3$, from 0 to 0.4% by weight of P, from 0 to 0.4% by weight of alkali (calculated as alkali metal) and $TiO_2$ in anatase form having a BET surface area of from 10 to 40 $m^2/g$ as balance on support material,
(iii) the most active catalyst comprises from 8 to 12% by weight, based on the total catalyst, of active composition comprising from 5 to 30% by weight of $V_2O_5$, from 0 to 3% by weight of $Sb_2O_3$, from 0.05 to 0.4% by weight of P, from 0 to 0.3% by weight of alkali (calculated as alkali metal) and $TiO_2$ in anatase form having a BET surface area of from 15 to 50 $m^2/g$ as balance on support material,

with the proviso that the BET surface area of the titanium dioxide in the uppermost layer (i) is less than the BET surface area of the titanium dioxide in the middle layer or layers (ii) and the BET surface area of the titanium dioxide in the bottom-most layer (iii) is greater than the BET surface area of the titanium dioxide in the middle layer or layers.

**2.** The catalyst system according to claim 1, wherein two middle layers (ii) are present and the titanium dioxide in the upper middle layer has a BET surface area of from 10 to 35 m$^2$/g and that in the lower middle layer has a BET surface area of from 15 to 40 m$^2$/g, with the proviso that the BET surface area of the titanium dioxide in the upper middle layer is less than or equal to the BET surface area in the lower middle layer.

**3.** The catalyst system according to claim 1 or 2, wherein the titanium dioxide in at least one catalyst layer consists of a mixture of titanium dioxides having different BET surface areas.

**4.** The catalyst system according to claim 3, wherein the mixture comprises a titanium dioxide having a BET surface area of from 5 to 15 m$^2$/g and a titanium dioxide having a BET surface area of from 15 to 50 m$^2$/g in a ratio of

(i) from 1:1.5 to 1:3 in the uppermost layer,
(iia) from 1:2 to 1:4 in the upper middle layer,
(iib) from 1:2.5 to 1:4 in the lower middle layer and
(iii) from 1:3 to 1:5 in the bottom-most layer.

**5.** The catalyst system according to any of claims 1 to 4, wherein the bed length

(i) of the uppermost catalyst layer is from 80 to 160 cm,
(iia) of the upper middle catalyst layer is from 20 to 60 cm,
(iib) of the lower middle catalyst layer is from 30 to 100 cm and
(iii) of the bottom-most catalyst layer is from 40 to 90 cm.

**6.** The catalyst system according to any of claims 1 to 5 which has four superposed layers, wherein

(i) the least active catalyst comprises from 7 to 10% by weight, based on the total catalyst, of active composition comprising from 6 to 11 % by weight of $V_2O_5$, from 0 to 3% by weight of $Sb_2O_3$, from 0.1 to 1 % by weight of alkali (calculated as alkali metal) and $TiO_2$ in anatase form having a BET surface area of from 5 to 30 m$^2$/g as balance on support material,
(iia) the next more active catalyst comprises from 7 to 12% by weight, based on the total catalyst, of active composition comprising from 4 to 15% by weight of $V_2O_5$, from 0 to 3% by weight of $Sb_2O_3$, from 0.1 to 1% by weight of alkali (calculated as alkali metal), from 0 to 0.4% by weight of P and $TiO_2$ in anatase form having a BET surface area of from 10 to 35 m$^2$/g as balance on support material,
(iib) the next more active catalyst comprises from 7 to 12% by weight, based on the total catalyst, of active composition comprising from 5 to 15% by weight of $V_2O_5$, from 0 to 3% by weight of $Sb_2O_3$, from 0 to 0.4% by weight of alkali (calculated as alkali metal), from 0 to 0.4% by weight of P and $TiO_2$ in anatase form having a BET surface area of from 15 to 40 m$^2$/g as balance on support material,
(iii) the most active catalyst comprises from 8 to 12% by weight, based on the total catalyst, of active composition comprising from 5 to 30% by weight of $V_2O_5$, from 0 to 3% by weight of $Sb_2O_3$, from 0.05 to 0.4% by weight of P and $TiO_2$ in anatase form having a BET surface area of from 15 to 50 m$^2$/g as balance on support material.

**7.** A process for preparing phthalic anhydride by gas-phase oxidation of xylene, naphthalene or mixtures thereof in a shell-and-tube reactor, wherein the starting materials are passed over a catalyst system according to any of claims 1 to 6.

**Revendications**

**1.** Système catalytique pour la préparation d'anhydride phtalique, qui présente au moins trois couches de catalyseur qui sont disposées les unes sur les autres dans un tube réactionnel et dont l'activité catalytique croît de couche en couche et dont la masse active contient 70 à 99 % en poids de dioxyde de titane dans la modification anatase, dans lequel

(i) le catalyseur le plus faiblement actif présente sur une matière de support 7 à 10 % en poids, par rapport au catalyseur total, de masse active contenant 6 à 11 % en poids de $V_2O_5$, 0 à 3 % en poids de $Sb_2O_3$, 0,1 à 1 % en poids d'alcali (calculé sous la forme de métal alcalin) et, comme reste, du $TiO_2$ sous forme d'anatase avec une surface spécifique BET de 5 à 30 m$^2$/g,
(ii) le catalyseur plus actif suivant présente sur une matière de support 7 à 12 % en poids, par rapport au catalyseur total, de masse active contenant 5 à 13 % en poids de $V_2O_5$, 0 à 3 % en poids de $Sb_2O_3$, 0 à 0,4 % en poids de P, 0 à 0,4 % en poids d'alcali (calculé sous la forme de métal alcalin) et, comme reste, du $TiO_2$ sous forme d'anatase avec une surface spécifique BET de 10 à 40 $m^2$/g,

(iii) le catalyseur le plus actif présente sur une matière de support 8 à 12 % en poids, par rapport au catalyseur total, de masse active contenant 5 à 30 % en poids de $V_2O_5$, 0 à 3 % en poids de $Sb_2O_3$, 0,05 à 0,4% en poids de P, 0 à 0,3 % en poids d'alcali (calculé sous la forme de métal alcalin) et, comme reste, du $TiO_2$ sous forme d'anatase avec une surface spécifique BET de 15 à 50 $m^2$/g,

à la condition que la surface spécifique BET du dioxyde de titane dans la couche supérieure (i) soit plus petite que la surface spécifique BET du dioxyde de titane dans la ou les couches centrales (ii) et que la surface spécifique BET du dioxyde de titane dans la couche inférieure (iii) soit plus grande que la surface spécifique BET du dioxyde de titane dans la ou les couches centrales.

2. Système catalytique suivant la revendication 1, dans lequel il y a deux couches centrales (ii) dont le dioxyde de titane présente dans la couche centrale supérieure une surface spécifique BET de 10 à 35 $m^2$/g et dans la couche centrale inférieure une surface spécifique BET de 15 à 40 $m^2$/g, à la condition que la surface spécifique BET du dioxyde de titane dans la couche centrale supérieure soit plus petite ou égale à la surface spécifique BET de la couche centrale inférieure.

3. Système catalytique suivant la revendication 1 ou 2, dans lequel le dioxyde de titane est constitué dans au moins une couche de catalyseur d'un mélange de dioxydes de titane de surfaces spécifiques BET différentes.

4. Système catalytique suivant la revendication 3, dans lequel le mélange est constitué d'un dioxyde de titane présentant une surface spécifique BET de 5 à 15 $m^2$/g et d'un dioxyde de titane présentant une surface spécifique BET de 15 à 50 $m^2$/g dans un rapport

(i) de 1/1,5 à 1/3 dans la couche supérieure,
(iia) de 1/2 à 1/4 dans la couche centrale supérieure,
(iib) de 1/2,5 à 1/4 dans la couche centrale inférieure, et
(iii) de 1/3 à 1/5 dans la couche inférieure.

5. Système catalytique suivant les revendications 1 à 4, dans lequel la longueur de garnissage

(i) de la couche de catalyseur supérieure est de 80 à 160 cm,
(iia) de la couche de catalyseur centrale supérieure est de de 20 à 60 cm,
(iib) de la couche de catalyseur centrale inférieure est de de 30 à 100 cm, et
(iii) de la couche de catalyseur inférieure est de 40 à de 90 cm.

6. Système catalytique suivant les revendications 1 à 5, qui présente quatre couches disposées les unes sur les autres, dans lequel

(i) le catalyseur le plus faiblement actif présente sur une matière de support 7 à 10 % en poids, par rapport au catalyseur total, de masse active contenant 6 à 11 % en poids de $V_2O_5$, 0 à 3 % en poids de $Sb_2O_3$, 0,1 à 1 % en poids d'alcali (calculé sous la forme de métal alcalin) et, comme reste, du $TiO_2$ sous forme d'anatase avec une surface spécifique BET de 5 à 30 $m^2$/g,

(iia) le catalyseur plus actif suivant présente sur une matière de support 7 à 12 % en poids, par rapport au catalyseur total, de masse active contenant 4 à 15 % en poids de $V_2O_5$, 0 à 3 % en poids de $Sb_2O_3$, 0,1 à 1 % en poids d'alcali (calculé sous la forme de métal alcalin), 0 à 0,4 % en poids de P et, comme reste, du $TiO_2$ sous forme d'anatase avec une surface spécifique BET de 10 à 35 $m^2$/g,

(iib) le catalyseur plus actif suivant présente sur une matière de support 7 à 12 % en poids, par rapport au catalyseur total, de masse active contenant 5 à 15 % en poids de $V_2O_5$, 0 à 3 % en poids de $Sb_2O_3$, 0 à 0,4 % en poids d'alcali (calculé sous la forme de métal alcalin), 0 à 0,4 % en poids de P et, comme reste, du $TiO_2$ sous forme d'anatase avec une surface spécifique BET de 15 à 40 $m^2$/g,

(iii) le catalyseur le plus actif présente sur une matière de support 8 à 12 % en poids, par rapport au catalyseur total, de masse active contenant 5 à 30 % en poids de $V_2O_5$, 0 à 3 % en poids de $Sb_2O_3$, 0,05 à 0,4 % en poids de P et, comme reste, du $TiO_2$ sous forme d'anatase avec une surface spécifique BET de 15 à 50 $m^2$/g.

7. Procédé de préparation d'anhydride phtalique par oxydation en phase gazeuse de xylène, de naphtalène ou de

EP 1 628 763 B1

leurs mélanges dans un réacteur à faisceau tubulaire, **caractérisé en ce que** les matières de départ sont conduites par l'intermédiaire d'un système catalytique suivant les revendications 1 à 6.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2238067 A
- DE 19823275 A
- WO 0370680 A
- EP 1063222 A
- WO 9817608 A
- DE 2106796 A
- EP 744214 A
- DE 19633757 A
- EP 522871 A
- DE 19824532 A
- DE 19807018 A
- DE 2005969 A